# EUROPÄISCHE PATENTSCHRIFT

(11) **EP 4 291 704 B1**
(45) Veröffentlichungstag und Bekanntmachung des Hinweises auf die Patenterteilung: **28.08.2024**
(21) Anmeldenummer: 23717404.0
(22) Anmeldetag: 31.03.2023
(51) Int. Cl.: D06P 1/44, D06P 5/00, D06P 1/52

(54) **VERFAHREN ZUM AUFBRINGEN VON ELASTOMER AUF BEIDE SEITEN EINER STOFFLAGE**
METHOD FOR APPLYING ELASTOMER ONTO BOTH SIDES OF A MATERIAL LAYER
PROCÉDÉ D'APPLICATION D'ÉLASTOMÈRE SUR LES DEUX CÔTÉS D'UNE COUCHE DE MATÉRIAU

(30) Priorität: 29.04.2022 DE 102022110520
(43) Veröffentlichungstag der Anmeldung: 20.12.2023
(73) Patentinhaber: NTT New Textile Technologies GmbH, 72336 Balingen (DE)
(72) Erfinder: BAUER, Hans, 73226 Balingen (DE)
(74) Vertreter: Müller, Gottfried
(86) Internationale Anmeldenummer: PCT/EP2023/025146
(87) Internationale Veröffentlichungsnummer: WO 2023/208411

(56) Entgegenhaltungen:
- EP-A1- 0 400 573
- EP-A1- 2 365 493
- DE-A1- 102017 119 328

## Beschreibung

Die Erfindung bezieht sich auf ein Verfahren zum Aufbringen von Elastomer auf beide Seiten einer Stofflage, beispielsweise einer Stofflage in einem Bekleidungsstück.

Bei einem aus der EP 1 211 956 B2 bekannten Verfahren zur Herstellung von Unterbekleidungsstücken wie Büstenhalter, Miederhosen, Sporthosen und dergleichen werden eine obere Lage und eine untere Lage des Unterbekleidungsstückes im Randbereich durch den Auftrag eines elastomeren Klebemittels miteinander verbunden, der im Siebdruckverfahren aufgebracht wird. Im noch nicht abgebundenen Zustand des Klebemittels wird die obere Lage des Bekleidungsstückes auf die untere Lage aufgelegt und angedrückt. Der Randbereich des Unterbekleidungsstückes wird durch die elastomeren Klebstoffverbindungen definiert. Außerhalb der Klebstoffverbindungen liegen die Lagen lose aufeinander.

Aus der DE 199 42 996 B4 ist ein Verfahren zur Herstellung eines Unterbekleidungsstückes mit einem mindestens einlagigen textilen Trägermaterial bekannt, bei dem auf einem einlagigen textilen Trägermaterial bereichsweise ein einseitiger Klebstoffauftrag aus einem elastomeren Klebstoff erfolgt, der nachfolgend mit einem Beflockungsmaterial beschichtet wird.

Aus der Druckschrift Joshi, M., Butola, B. S.: Application technologies for coating lamination and finishing textiles. In: Advances in the dyeing and finishing of technical textiles. Oxford: Woodhead Publishing, 2013 (Woodhead Publishing series in textiles) - ISBN 978-0-85709-433-9 ist es bekannt, ein Gummiband über zwei rotierende Walzen zu legen und über das Gummiband eine Stofflage zu führen, welche mittels einer Rakel beschichtet wird. Das Gummiband kann sich aufgrund seiner Elastizität im Bereich zwischen den rotierenden Walzen geringfügig dehnen und nachgeben, wenn die Rakel über die Stofflage streicht.

Aus der EP 2 365 493 A1 ist ein Verfahren zur Herstellung eines flexiblen PTC-Heizelements bekannt. Bei dem Verfahren wird eine PTC-Paste, die als Bindemittel ein Polymer enthalten kann, durch einen Stoff mit gitterförmiger Struktur gepresst, der auf einem nachgiebigen Substrat, beispielsweise ein geschäumtes Polyurethan, aufliegt. Das Substrat wirkt als Sperre für die PTC-Paste.

Der Erfindung liegt die Aufgabe zugrunde, mit einfachen und effizient durchzuführenden Maßnahmen eine Stofflage auf beiden Seiten mit Elastomer zu beschichten.

Diese Aufgabe wird erfindungsgemäß mit den Merkmalen des Anspruches 1 gelöst. Die Unteransprüche geben zweckmäßige Weiterbildungen an.

Mithilfe des erfindungsgemäßen Verfahrens kann eine Stofflage auf beiden Seiten mit Elastomer beschichtet werden, wobei die Stofflage für ein Bekleidungsstück, beispielsweise ein Unterbekleidungsstück oder ein Oberbekleidungsstück eingesetzt wird. Die Stofflage kommt zum Beispiel bei Büstenhaltern, Slips, Sporthosen oder T-Shirts zum Einsatz. In Betracht kommt auch eine Verwendung der Stofflage für eine Gelenkbandage, beispielsweise eine Knie- oder Ellbogenbandage.

Bei dem Verfahren wird Elastomer in einer einfachen und effizient durchzuführenden Weise auf beide Seiten einer Stofflage aufgebracht. Das Material der Stofflage weist vorzugsweise eine offene Struktur auf und ist beispielsweise als Tüll oder Mesh ausgeführt. Zunächst wird die Stofflage in einem ersten Schritt auf eine nachgiebige Unterlage aufgelegt. Im nächsten Schritt wird Elastomer im fließfähigen Zustand mit Druck und ausschließlich von einer Seite auf die Stofflage aufgebracht. Der Druck wird hierbei so gewählt, dass die nachgiebige Unterlage, auf der die Stofflage aufliegt, während des Aufbringens des Elastomers um mindestens 1% und maximal 50% zusammengedrückt wird. Aufgrund der Nachgiebigkeit der Unterlage, auf der die Stofflage aufliegt, durchdringt das von oben mit Druck applizierte Elastomer die Stofflage vollständig und erreicht die gegenüberliegende, der nachgiebigen Unterlagen zugewandte Seite der Stofflage. Der Druck, unter dem das Elastomer aufgebracht wird, drückt die nachgiebige Unterlage zusammen, was die Ausbreitung des Elastomers innerhalb der Stofflage bis zum Erreichen der der Unterlage zugewandten Seite unterstützt und erleichtert.

Es reicht aus, dass das Elastomer ausschließlich von oben auf die Stofflage aufgebracht und unter Druck gesetzt wird, so dass ein einziger Prozessschritt zum Aufbringen des Elastomers genügt. Es ist nicht erforderlich, in einem zweiten Prozessschritt zusätzlich Elastomer auf die Stofflage aufzubringen.

Der Druck, mit dem das Elastomer auf die Stofflage aufgebracht wird, drückt die elastisch nachgiebige Unterlage teilweise zusammen. Die nachgiebige Unterlage wird durch den Druck während des Aufbringens des Elastomers um mindestens 1% und maximal 50% zusammengedrückt, wobei gegebenenfalls auch ein Zusammendrücken von mindestens 5%, mindestens 10%, mindestens 15%, mindestens 20% oder ein noch höherer Wert bis und maximal 50% möglich ist.

Dementsprechend wird der Druck an die Nachgiebigkeit der Unterlage angepasst.

Der Druck, mit dem das Elastomer im fließfähigen Zustand auf die Stofflage aufgebracht wird, wird außerdem so gewählt, dass die nachgiebige Unterlage nur um ein maximales Maß von 50% zusammengedrückt wird, beispielsweise um maximal 40% oder maximal 30%. Die Begrenzung des Drucks auf einen Maximalwert gewährleistet eine rasche Durchführung des Prozessschritts und außerdem ein gleichmäßiges Aufbringen des Elastomers.

Nach dem Aufbringen des Elastomers kann in einem weiteren, nachfolgenden Prozessschritt im noch nicht abgebundenen Zustand des Elastomers auf mindestens eine Seite der Stofflage ein Beflockungsmaterial aufgebracht werden. Dies erfolgt beispielsweise dadurch, dass nach dem Aufbringen des Elastomers die obenliegende Seite mit Beflockungsmaterial bestreut wird, das sich mit dem noch nicht abgebundenen Elastomer verbindet und nach dem Aushärten oder Trocknen des Elastomers eine feste Verbindung zum Elastomer sowie zur Stofflage aufweist. Das Beflockungsmaterial ist hautsympatisch und verbessert den Tragekomfort und ist außerdem in der Lage, Feuchtigkeit aufzunehmen. Da Beflockungsmaterial besteht bevorzugt aus Naturfasern, synthetischen Fasern oder halbsynthetischen Fasern.

Gegebenenfalls werden beide Seiten der Stofflage mit Beflockungsmaterial versehen. Hierzu wird die Stofflage, nachdem das Beflockungsmaterial auf die Oberseite aufgebracht wurde, von der nachgiebigen Unterlagen abgenommen und anschließend die Unterseite mit dem Beflockungsmaterial versehen.

Das Elastomer kann entweder flächig oder gemäß eines Musters auf die Stofflage aufgebracht werden.

Die nachgiebige Unterlage weist insbesondere eine ebene, geradflächig Oberfläche auf, auf die die Stofflage aufgelegt wird. Es kann zweckmäßig sein, dass die nachgiebige Unterlage beim Aufbringen des Elastomers gleichmäßig zusammengedrückt wird. Die nachgiebige Unterlage ist vorzugsweise als Quader ausgeführt, beispielsweise als ein quaderförmiger Block.

Als nachgiebige Unterlage, auf die die Stofflage aufgelegt wird, wird eine Schaumstofflage verwendet. Grundsätzlich kommen Unterlagen in Betracht, die aus einem elastisch-nachgiebigen Material gefertigt sind.

Auf die nachgiebige Unterlage wird eine reibungsreduzierte Zusatzschicht aufgebracht, auf die die Stofflage aufgelegt wird. Die reibungsreduzierte Zusatzschicht verhindert ein Festkleben des Elastomers, das die Stofflage durchdringt und sich bis zu der der nachgiebigen Unterlage zugewandten Seite der Stofflage ausbreitet. Als Zusatzschicht wird beispielsweise eine Teflonschicht verwendet.

Die reibungsreduzierte Zusatzschicht kann als separates Bauteil ausgeführt sein, das auf die nachgiebige Unterlage aufgelegt und gegebenenfalls auf der Unterlage fixiert wird. Alternativ ist es auch möglich, dass die reibungsreduzierte Zusatzschicht fest mit der nachgiebigen Unterlage verbunden ist und die Oberseite der nachgiebigen Unterlage bildet, auf die die Stofflage aufgelegt wird.

Das erfindungsgemäße Verfahren, bei dem Elastomer auf beide Seiten einer Stofflage aufgebracht wird, weist folgende Verfahrensschritte auf:
- die Stofflage wird auf eine nachgiebige Unterlage aufgelegt,
- das Elastomer wird im fließfähigen Zustand mit Druck auf die Stofflage aufgebracht wird, wobei das Elastomer ausschließlich von einer Seite auf die Stofflage aufgebracht und die nachgiebige Unterlage während des Aufbringens des Elastomers (3) unter dem Druck des Elastomers um mindestens 1% und maximal 50% zusammengedrückt wird,
- als nachgiebige Unterlage, auf die die Stofflage aufgelegt wird, wird eine Schaumstofflage verwendet,
- auf der Schaumstofflage befindet sich eine reibungsreduzierte Zusatzschicht,
- das von oben mit Druck applizierte Elastomer durchdringt die Stofflage vollständig und erreicht die gegenüberliegende, der nachgiebigen Unterlage zugewandte Seite der Stofflage.

Somit bezieht sich die Erfindung auf ein Verfahren zum Aufbringen von Elastomer auf beide Seiten einer Stofflage, bei dem die Stofflage auf eine nachgiebige Unterlage aufgelegt und das Elastomer im fließfähigen Zustand mit Druck auf die Stofflage aufgebracht wird, wobei das Elastomer ausschließlich von einer Seite auf die Stofflage aufgebracht und die nachgiebige Unterlage während des Aufbringens des Elastomers unter dem Druck des Elastomers um mindestens 1% und maximal 50% zusammengedrückt wird. Als nachgiebige Unterlage, auf die die Stofflage aufgelegt wird, wird eine Schaumstofflage verwendet, auf der sich eine reibungsreduzierte Zusatzschicht befindet. Das von oben mit Druck applizierte Elastomer durchdringt die Stofflage vollständig und erreicht die gegenüberliegende, der nachgiebigen Unterlage zugewandte Seite der Stofflage.

In einer weiteren vorteilhaften Ausführung wird das Elastomer im Siebdruckverfahren auf die Stofflage aufgebracht. Hierbei wird ein Siebdrucksieb auf die Stofflage aufgelegt und Elastomer im fließfähigen Zustand auf das Siebdrucksieb aufgegeben. Daraufhin wird eine Rakel über das Siebdrucksieb geführt, die einen Druck auf das Elastomer ausübt und das Elastomer durch das Siebdrucksieb hindurch und in die unter dem Siebdrucksieb liegende Stofflage hineinpresst. Das Siebdrucksieb kann entweder gleichmäßig verteilte Sieböffnungen aufweisen oder als eine Schablone ausgebildet sein, in die Ausnehmungen in Form eines Musters eingebracht sind, durch die das Elastomer von der Rakel hindurchgepresst wird und in der Stofflage ein entsprechendes Muster bildet.

Als Elastomer wird ein Silikon, beispielsweise ein thermoplastisches Silikon verwendet, das bei Erwärmung weich oder flüssig wird und mit dem Abkühlen aushärtet. Im erwärmten Zustand kann das Elastomer über die Düse aufgetragen werden, woraufhin das Elastomer aushärtet und hierbei die feste Verbindung mit der Stofflage eingeht und außerdem das Kabel schützt und an der Stofflage sichert. Im ausgehärteten Zustand besitzt das Elastomer ein elastisches Verhalten. Als Elastomer wird ein Silikon verwendet.

Es kann genügen, nur eine einzige Stofflage einzusetzen, auf die Elastomer im fließfähigen Zustand mit Druck aufgebracht wird, wobei sich das Elastomer bis zur gegenüberliegenden Seite ausbreitet. In einer weiteren vorteilhaften Ausführung ist es auch möglich, mehrere Stofflagen, vorteilhafterweise zwei Stofflagen unmittelbar aufeinanderzulegen, wobei auf die obere oder oberste Stofflage Elastomer im fließfähigen Zustand mit Druck aufgebracht wird und sich das Elastomer bis zur gegenüberliegenden Seite dieser Stofflage sowie durch die darunterliegende Stofflage ausbreitet, bis das Elastomer die untere Seite der darunterliegenden Stofflage erreicht hat.

Des Weiteren kann ein Bekleidungsstück oder eine Bandage mit einer Stofflage durch das vorbeschriebenen Verfahren hergestellt werden. Das Bekleidungsstück oder die Bandage kann entweder ausschließlich aus einer derartigen Stofflage oder mehreren derartigen Stofflagen hergestellt sein oder zusätzlich zu einer oder mehreren derartigen Stofflagen auch weitere Stofflagen aufweisen, die einen sich unterscheidenden Aufbau aufweisen. Weitere Vorteile und zweckmäßige Ausführungen sind den weiteren Ansprüchen, der Figurenbeschreibung und den Zeichnungen zu entnehmen. Es zeigen:
- Fig. 1: einen Schnitt durch eine Vorrichtung zum Aufbringen von Elastomer auf beide Seiten einer Stofflage, dargestellt zu Beginn des Verfahrens,
- Fig. 2: eine Darstellung gemäß Fig. 1, jedoch zu einem fortgeschrittenen Zeitpunkt des Verfahrens.

In den Figuren sind gleiche Bauteile mit gleichen Bezugszeichen versehen.

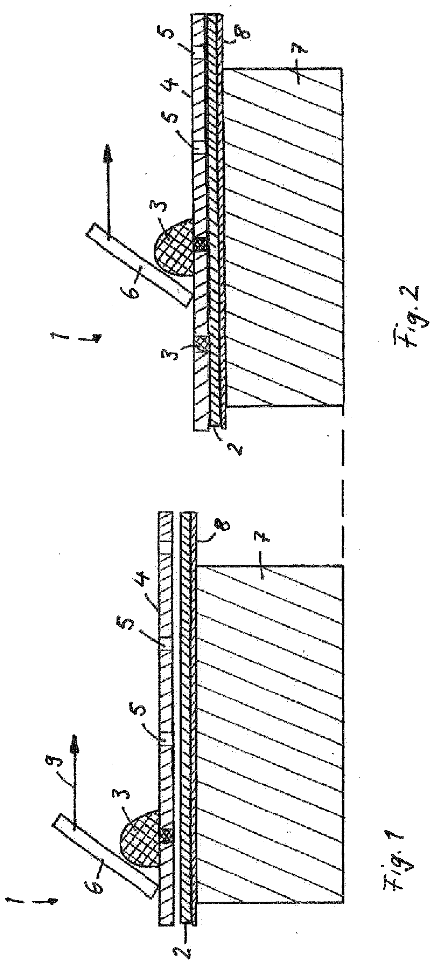

In Fig. 1 ist eine Vorrichtung 1 zum Aufbringen von Elastomer 3 auf die beiden gegenüberliegenden Seiten einer Stofflage 2 dargestellt. Bei der Vorrichtung 1 handelt es sich um eine Siebdruckvorrichtung, bei der das Elastomer 3 im fließfähigen, noch nicht ausgehärteten Zustand mithilfe eines Siebdrucksiebs 4 und einer Rakel 6 von oben auf die Stofflage 2 aufgebracht wird. Hierfür wird das Elastomer 3 auf das Siebdrucksieb 4 aufgebracht und die Rakel 6 in Horizontalrichtung gemäß Pfeil 9 über das Siebdrucksieb 4 bewegt. Die Rakel 6 steht gegenüber der Horizontalebene schräg, der Winkel zwischen der Rakel 6 und der Horizontalebene liegt in einem Winkelbereich von 45° bis 60°, so dass das Elastomer 3 zum einen ebenfalls in Horizontalrichtung über das Siebdrucksieb 4 bewegt wird, zum andern aber auch eine nach unten gerichtete Vertikalkraft erfährt und mit Druck durch das Siebdrucksieb 4 hindurchgepresst wird.

Die Stofflage 2 liegt auf einer nachgiebigen Unterlage 7 auf, die als Schaumstofflage in Form eines elastisch-nachgiebigen Schaumstoffblocks 7 ausgeführt ist. Auf dem Schaumstoffblock 7 befindet sich eine reibungsarme Zusatzschicht 8, die beispielsweise als eine Teflonschicht ausgebildet ist und auf der die mit dem Elastomer zu behandelnde Stofflage 2 aufliegt. Aufgrund der Nachgiebigkeit kann der Schaumstoffblock 7 zusammengepresst werden, sobald bei einer Bewegung der Rakel 6 in Pfeilrichtung 9 das Elastomer 3 mit einer vertikal nach unten weisenden, in Richtung auf die Stofflage 2 zeigenden Druckkomponente beaufschlagt wird.

Wenn die Rakel 6 in Pfeilrichtung 9 über das Siebdrucksieb 4 bewegt wird, wird das Elastomer 3, das sich auf dem Siebdrucksieb 4 befindet, durch Ausnehmungen 5 im Siebdrucksieb 4 nach unten in Richtung auf die darunterliegende Stofflage 2 gedrückt und breitet sich durch die Poren der Stofflage 2 aus. Das Elastomer 3 dringt, ausgehend von der obenliegenden Seite der Stofflage 2, durch die Stofflage 2 bis zur gegenüberliegenden, dem Schaumstoffblock 7 zugewandten untenliegenden Seite der Stofflage 2 hindurch, so dass sowohl die obenliegende Seite der Stofflage 2 als auch die untenliegende Seite der Stofflage 2 mit Elastomer 3 benetzt sind.

Aufgrund des elastisch-nachgiebigen Verhaltens des Schaumstoffblocks 7 wird bei einer Bewegung der Rakel 6 über das Siebdrucksieb 4 und der hierbei entstehenden, nach unten gerichteten Vertikalkraft der Schaumstoffblock 7 zusammengedrückt, was aus einem Vergleich von Fig. 1 und Fig. 2 ersichtlich ist. Die Parameter der Vorrichtung 1 im Hinblick auf die Schrägstellung der Rakel 6, die Größe der Ausnehmungen 5 im Siebdrucksieb 4, den verwendeten Stoff der Stofflage 2, die Elastizität des Schaumstoffblocks 7 und die Viskosität des Elastomers 3 können ebenso wie Prozessparameter im Hinblick auf die Verfahrgeschwindigkeit der Rakel 6 in der Weise aufeinander abgestimmt werden, dass der Schaumstoffblock 7 in einem gewünschten Maß zusammengedrückt wird. Das Zusammendrücken des Schaumstoffblocks 7 liegt beispielsweise in einem Bereich zwischen 1% und 50%, bezogen auf die Höhe des Schaumstoffblocks 7 im unbelasteten Zustand.

Das vertikale Zusammendrücken des Schaumstoffblocks 7 während der Horizontalbewegung der Rakel 7 gemäß Pfeil 9 unterstützt und verbessert die Ausbreitung des Elastomers 3 von der obenliegenden Seite zur untenliegenden Seite der Stofflage 2. Durch den Druck des Elastomers 3 weicht der Schaumstoffblock 7 nach unten aus, so dass das Elastomer 3 geringfügig über die untenliegenden Seite der Stofflage 2 hinaustreten kann. Gegebenenfalls wird Elastomer, das sich unterhalb der untenliegenden Seite der Stofflage 2 befindet, durch die Rückfederbewegung des Schaumstoffblocks 7 wieder in die Stofflage 2 zurückgedrängt. In jedem Fall ist aber gewährleistet, dass das Elastomer die untenliegenden Seite der Stofflage 2 erreicht.

Je nach Ausführung des Siebdrucksiebs 4 können verschiedenartige Elastomermuster an der obenliegenden Seite und der untenliegenden Seite der Stofflage 2 erzeugt werden. Es ist ein flächiger, linienförmiger und/oder punktförmiger Elastomerauftrag möglich.

Nach Beendigung des Siebdruckvorgangs kann die Stofflage 2 an einer Seite oder an beiden Seiten beflockt werden. Hierfür wird noch vor dem Aushärten des Elastomers 3 ein Beflockungsmaterial auf die obenliegende Seite der Stofflage 2 aufgebracht. Gegebenenfalls kann auch auf die untenliegende Seite der Stofflage 2 mit Beflockungsmaterial versehen werden, wofür die Stofflage 2 beispielsweise in der Vorrichtung 1 gewendet wird, so dass die zuvor unten liegende Seite nach oben weist und mit Beflockungsmaterial bestreut werden kann. Nach dem Aushärten des Elastomers 3 ist das Beflockungsmaterial über das Elastomer 3 fest mit einer oder mit beiden Seiten der Stofflage 2 verbunden.

## Patentansprüche

1. Verfahren zum Aufbringen von Elastomer (3) auf beide Seiten einer Stofflage (2) für ein Bekleidungsstück oder eine Gelenkbandage, bei dem die Stofflage (2) auf eine nachgiebige Unterlage (7) aufgelegt und das Elastomer (3) im fließfähigen Zustand mit Druck auf die Stofflage (2) aufgebracht wird, wobei das Elastomer (3) ausschließlich von einer Seite auf die Stofflage (2) aufgebracht wird und die nachgiebige Unterlage (7) während des Aufbringens des Elastomers (3) unter dem Druck des Elastomers (3) zusammengedrückt wird, **dadurch**
**gekennzeichnet,**
**dass** als nachgiebige Unterlage (7), auf die die Stofflage (2) aufgelegt wird, eine Schaumstofflage (7) verwendet wird, auf der sich eine reibungsreduzierte Zusatzschicht (8) befindet, und dass das von oben mit Druck applizierte Elastomer (3) die Stofflage (2) vollständig durchdringt und die gegenüberliegende, der nachgiebigen Unterlage (7) zugewandte Seite der Stofflage (2) erreicht, wobei als Elastomer (3) ein Silikon verwendet wird, wobei die nachgiebige Unterlage (7) durch den Druck während des Aufbringens des Elastomers (3) um mindestens 1% und maximal 50% zusammengedrückt wird.

2. Verfahren nach Anspruch 1,
**dadurch gekennzeichnet,**
**dass** nach dem Aufbringen des Elastomers (3) im noch nicht abgebundenen Zustand des Elastomers (3) auf mindestens eine Seite der Stofflage (2), vorzugsweise auf beide Seiten der Stofflage (2) ein Beflockungsmaterial aufgebracht wird.

3. Verfahren nach Anspruch 1 oder 2,
**dadurch gekennzeichnet,**
**dass** als Zusatzschicht (8) eine Teflonschicht (8) verwendet wird.

4. Verfahren nach einem der Ansprüche 1 bis 3,
**dadurch gekennzeichnet,**
**dass** das Elastomer (3) im Siebdruckverfahren auf die Stofflage (2) aufgebracht wird.

5. Verfahren nach einem der Ansprüche 1 bis 4,
**dadurch gekennzeichnet,**
**dass** auf genau eine Stofflage (2) Elastomer (3) im fließfähigen Zustand mit Druck aufgebracht wird

6. Verfahren nach einem der Ansprüche 1 bis 5,
**dadurch gekennzeichnet,**
**dass** zwei Stofflagen (2) unmittelbar aufeinanderliegen, wobei auf die obere Stofflage (2) Elastomer (3) im fließfähigen Zustand mit Druck aufgebracht wird.

## Claims

1. Method for applying elastomer (3) to both sides of a fabric layer (2) for a clothing piece or a joint bandage in which the fabric layer (2) is placed on a resilient support (7) and the elastomer (3) is applied in a flowable state under pressure to the fabric layer (2), wherein the elastomer (3) is applied to the fabric layer (2) exclusively from one side thereof and, during the application of the elastomer under pressure, the resilient support (7) is compressed, **characterized in that** the resilient support (7) onto which the fabric layer (2) is placed is a foam material layer (7) on which a low friction additional layer (8) is provided and that the elastomer (3), which is applied from above under pressure, completely penetrates the fabric layer (2) and reaches the opposite side of the fabric layer 2) facing the resilient support (7), wherein as elastomer (3) a silicon is used and wherein the resilient support (7) is compressed by the pressure during the application of the elastomer (3) by at least 1% and maximally 50%.

2. Method according to claim 1, **characterized in that** after the application of the elastomer (3) before curing of the elastomer (3) a flocking material is applied to at least one side of the fabric layer (2), preferably to both sides of the fabric layer (2).

3. Method according to claim 1 or 2, **characterized in that** the additional layer (8) is a Teflon layer.

4. Method according to any of claims 1 to 3, **characterized in that** the elastomer (3) is applied to the fabric layer (2) by a screen printing process.

5. Method according to any of claims 1 to 4, **characterized in that** elastomer (3) is applied under pressure in a flowable state to exactly one fabric layer (2).

6. Method according to any of claims 1 to 5, **characterized in that** two fabric layers (2) are disposed directly on top of one another and elastomer (3) is applied in a flowable state under pressure onto the upper fabric layer (2).

## Revendications

1. Procédé d'application d'élastomère (3) sur les deux faces d'une couche de tissu (2) pour un vêtement ou un bandage articulaire, dans lequel la couche de tissu (2) est posée sur un support souple (7) et l'élastomère ( 3) est dans un état fluide. Une pression est appliquée sur la couche de tissu (2), l'élastomère (3) étant appliqué sur la couche de tissu (2) exclusivement d'un côté et la base élastique (7) étant sous la pression de l'élastomère (3) lors de l'application de l'élastomère (3) est comprimé, **caractérisé en ce que**
comme support flexible (7) on utilise une couche de mousse (7) sur laquelle est posée la couche de matériau (2), sur laquelle se trouve une couche supplémentaire (8) à frottement réduit, et que l'élastomère (3) appliqué avec pression d'en haut La couche de matériau (2) pénètre complètement et atteint le côté opposé de la couche de matériau (2) faisant face à la base flexible (7), un silicone étant utilisé comme élastomère (3), la base flexible (7) étant affectée par la pression lors de l'application de l'élastomère (3) est comprimé d'au moins 1% et d'un maximum de 50%.

2. Procédé selon la revendication 1,
**caractérisé en ce que**
après que l'élastomère (3) a été appliqué dans l'état non encore pris de l'élastomère (3), un matériau de flocage est appliqué sur au moins un côté de la couche de tissu (2), de préférence sur les deux côtés de la couche de tissu ( 2).

3. Procédé selon la revendication 1 ou 2,
**Caractérisé en ce**
**qu'**une couche de Téflon (8) est utilisée comme couche supplémentaire (8).

4. Procédé selon l'une des revendications 1 à 3,
**caractérisé en ce que**
l'élastomère (3) est appliqué sur la couche de tissu (2) par un procédé de sérigraphie.

5. Procédé selon l'une des revendications 1 à 4,
**caractérisé en ce que**
l'élastomère (3) à l'état fluide est appliqué avec pression sur exactement une couche du matériau (2).

6. Procédé selon l'une des revendications 1 à 5,
**Caractérisé en ce que**
deux couches de matériau (2) sont directement superposées, un élastomère (3) étant appliqué sous pression sur la couche supérieure de matériau (2) à l'état fluide.
